(19) Europäisches Patentamt
European Patent Office
Office européen des brevets

(11) **EP 4 685 920 A1**

(12) **EUROPEAN PATENT APPLICATION**
published in accordance with Art. 153(4) EPC

(43) Date of publication:
**28.01.2026 Bulletin 2026/05**

(21) Application number: **24784887.2**

(22) Date of filing: **01.04.2024**

(51) International Patent Classification (IPC):
*H01M 10/48* [(2006.01)]    *G01R 31/367* [(2019.01)]
*G01R 31/382* [(2019.01)]    *G01R 31/385* [(2019.01)]
*H02J 7/00* [(2026.01)]

(52) Cooperative Patent Classification (CPC):
Y02E 60/10

(86) International application number:
**PCT/JP2024/013484**

(87) International publication number:
**WO 2024/210102 (10.10.2024 Gazette 2024/41)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(30) Priority: **03.04.2023 JP 2023060109**

(71) Applicant: **Maxell, Ltd.**
**Kyoto 618-8525 (JP)**

(72) Inventors:
• **KISHIMI, Yuko**
**Otokuni-gun, Kyoto 618-8525 (JP)**
• **MATSUMOTO, Nobuaki**
**Otokuni-gun, Kyoto 618-8525 (JP)**

(74) Representative: **Mewburn Ellis LLP**
**Aurora Building**
**Counterslip**
**Bristol BS1 6BX (GB)**

(54) **SECONDARY BATTERY DIAGNOSTIC METHOD, SECONDARY BATTERY DIAGNOSTIC PROGRAM, AND SECONDARY BATTERY DIAGNOSTIC DEVICE**

(57)    A method of diagnosing a secondary battery is provided that is capable of estimating, in a simple manner, electrolyte diffusion coefficient, which is one of the characteristic values of a secondary battery (i.e., characteristic parameters), without disassembling the secondary battery. A method of diagnosing a secondary battery includes: estimating an electrolyte diffusion coefficient at a time of diagnosis of a secondary battery being diagnosed, Dn, using a first trained model adapted to estimate the electrolyte diffusion coefficient of the secondary battery based on input data containing the following items, (1) to (6): (1) the rated capacity (initial capacity) of the secondary battery; (2) the discharge capacity measured at a discharge rate equivalent to that for the measurement of the rated capacity; and (3) the magnitude of electric current for measurements of the following items, (4) to (6): (4) the voltage drop as determined at a predetermined period of time from initiation of discharge (or voltage rise as determined at a predetermined period of time from initiation of charge); (5) the voltage drop as determined at a predetermined SOC; and (6) the temperature rise as determined at a predetermined SOC.

*Fig.5*

```
         START
           │
ESTIMATE Dn USING TRAINED MODEL A    ~S1
           │
ESTIMATE Dth USING TRAINED MODEL B   ~S2
           │
CALCULATE ΔD = Dn − Dth              ~S3
           │
          END
```

**Description**

TECHNICAL FIELD

The present invention relates to a method of diagnosing a secondary battery, a program for diagnosing a secondary battery, and an apparatus for diagnosing a secondary battery.

BACKGROUND ART

A secondary battery such as a lithium ion battery gradually decreases in discharge capacity with repeated charging and discharging. As such, the secondary battery is preferably diagnosed at an appropriate time to evaluate a degree of deterioration thereof and determine whether reuse is possible or determine a replacement period. Further, to reuse the secondary battery after diagnosis, preferably the diagnosis is performed in a non-destructive manner.

JP 2017-97997 A describes a characteristic analysis method for a secondary battery in which a model equation, parameters of which are characteristic values of members constituting the battery, is used, and the characteristic values of the member are estimated by fitting a voltage value of the battery represented by the model equation with actual measurement data. In this characteristic analysis method, the actual measurement data used is obtained by applying a charge/discharge pattern to the battery under analysis, which includes an operation period consisting of either a constant current discharge period or a constant current charge period, followed by a rest period.

Further, the publication describes, as the characteristic values estimated by the fitting with the actual measurement data, a lithium-ion diffusion coefficient in a positive-electrode active material, a lithium-ion diffusion coefficient in a negative-electrode active material, a lithium-ion diffusion coefficient in an electrolyte (electrolyte diffusion coefficient), an interface resistance in the positive-electrode active material, an interface resistance in the negative-electrode active material, a lithium-ion salt concentration in the electrolyte, and the like.

PRIOR ART DOCUMENTS

PATENT DOCUMENTS

Patent Document 1: JP 2017-97997 A

SUMMARY OF THE INVENTION

PROBLEMS TO BE SOLVED BY THE INVENTION

In the related art, the degree of deterioration of a secondary battery is evaluated on the basis of a magnitude of discharge capacity or a magnitude of internal resistance at the time of diagnosis. However, according to research conducted by the present inventors, it has been found that, even if the magnitudes of discharge capacity and the magnitudes of internal resistance at the time of diagnosis are substantially the same, the numbers of times that secondary batteries can be used thereafter are not necessarily substantially the same. Specifically, among secondary batteries having substantially the same magnitudes of discharge capacity and magnitudes of internal resistance at the time of diagnosis, there exist secondary batteries in which the discharge capacity drops sharply after a small number of charge/discharge cycles, secondary batteries in which the discharge capacity does not substantially drop even after charging/discharging, and the like. As a result, the remaining life of a secondary battery cannot be accurately evaluated by measuring only the magnitude of discharge capacity and the magnitude of internal resistance at the time of diagnosis.

JP 2017-97997 A above describes a method for estimating characteristic values of members constituting a secondary battery without disassembling the secondary battery. This method estimates the characteristic values of the members constituting the secondary battery through simulation. This simulation requires a certain amount of time and also requires one to master the use of software used for the simulation. Thus, it will be preferable if characteristic values can be estimated in a simpler manner. Further, this publication does not describe a specific method for evaluating the remaining life of the secondary battery from these characteristic values.

An object of the present invention is to provide a method of diagnosing a secondary battery, a program for diagnosing a secondary battery, and an apparatus for diagnosing a secondary battery capable of estimating, in a simple manner, electrolyte diffusion coefficient, which is one of the characteristic values of a secondary battery (i.e., characteristic parameters), without disassembling the secondary battery. Another object of the present invention is to provide a method of diagnosing a secondary battery, a program for diagnosing a secondary battery, and an apparatus for diagnosing a secondary battery, capable of evaluating remaining life more accurately than conventional techniques.

MEANS FOR SOLVING THE PROBLEMS

A method of diagnosing a secondary battery according to one embodiment of the present invention includes: estimating an electrolyte diffusion coefficient at a time of diagnosis of a secondary battery being diagnosed, Dn, using a first trained model adapted to estimate the electrolyte diffusion coefficient of the secondary battery based on input data containing the following items, (1) to (6):

(1) a rated capacity (initial capacity) of the secondary battery;
(2) a discharge capacity measured at a discharge rate equivalent to that for a measurement of the rated capacity; and
(3) a magnitude of electric current for measurements of the following items, (4) to (6):
(4) a voltage drop as determined at a predetermined period of time from initiation of discharge (or a voltage rise as determined at a predetermined period of time from initiation of charge);
(5) a voltage drop as determined at a predetermined SOC; and
(6) a temperature rise as determined at a predetermined SOC.

A method of diagnosing a secondary battery according to one embodiment of the present invention includes: estimating a difference ΔD for a secondary battery being diagnosed using a fifth trained model adapted to estimate the difference ΔD between an electrolyte diffusion coefficient of the secondary battery and a threshold value of electrolyte diffusion coefficient, that is the value at which the secondary battery becomes unsuitable for reuse, based on input data containing items (1) to (8) provided below:

(1) a rated capacity (initial capacity) of the secondary battery;
(2) a discharge capacity measured at a discharge rate equivalent to that for a measurement of the rated capacity; and
(3) a magnitude of electric current from measurements of the following items, (4) to (6):
(4) a voltage drop as determined at a predetermined period of time from initiation of discharge (or a voltage rise as determined at a predetermined period of time from initiation of charge);
(5) a voltage drop as determined at a predetermined SOC;
(6) a temperature rise as determined at a predetermined SOC;
(7) an electrode area; and
(8) a positive-electrode application quantity.

A program for diagnosing a secondary battery according to one embodiment of the present invention causes a computer to perform: estimating an electrolyte diffusion coefficient at a time of diagnosis of a secondary battery being diagnosed, Dn, using a first trained model adapted to estimate the electrolyte diffusion coefficient of the secondary battery based on input data containing the following items, (1) to (6):

(1) a rated capacity (initial capacity) of the secondary battery;
(2) a discharge capacity measured at a discharge rate equivalent to that for a measurement of the rated capacity; and
(3) a magnitude of electric current for measurements of the following items, (4) to (6):
(4) a voltage drop as determined at a predetermined period of time from initiation of discharge (or a voltage rise as determined at a predetermined period of time from initiation of charge);
(5) a voltage drop as determined at a predetermined SOC; and
(6) a temperature rise as determined at a predetermined SOC.

A program for diagnosing a secondary battery according to one embodiment of the present invention causes a computer to perform: estimating a difference ΔD for a secondary battery being diagnosed using a fifth trained model adapted to estimate the difference ΔD between an electrolyte diffusion coefficient of the secondary battery and a threshold value of electrolyte diffusion coefficient, that is the value at which the secondary battery becomes unsuitable for reuse, based on input data containing items (1) to (8) provided below:

(1) a rated capacity (initial capacity) of the secondary battery;
(2) a discharge capacity measured at a discharge rate equivalent to that for a measurement of the rated capacity; and
(3) a magnitude of electric current for measurements of the following items, (4) to (6):
(4) a voltage drop as determined at a predetermined period of time from initiation of discharge (or a voltage rise as determined at a predetermined period of time from initiation of charge);
(5) a voltage drop as determined at a predetermined SOC;
(6) a temperature rise as determined at a predetermined SOC;
(7) an electrode area; and

(8) a positive-electrode application quantity.

An apparatus for diagnosing a secondary battery according to one embodiment of the present invention includes: an estimation device adapted to estimate an electrolyte diffusion coefficient at a time of diagnosis of a secondary battery being diagnosed, Dn, using a first trained model adapted to estimate the electrolyte diffusion coefficient of the secondary battery based on input data containing the following items, (1) to (6):

(1) a rated capacity (initial capacity) of the secondary battery;
(2) a discharge capacity measured at a discharge rate equivalent to that for a measurement of the rated capacity; and
(3) a magnitude of electric current for measurements of the following items, (4) to (6):
(4) a voltage drop as determined at a predetermined period of time from initiation of discharge (or a voltage rise as determined at a predetermined period of time from initiation of charge);
(5) a voltage drop as determined at a predetermined SOC; and
(6) a temperature rise as determined at a predetermined SOC.

An apparatus for diagnosing a secondary battery according to one embodiment of the present invention includes: an estimation device adapted to estimate a difference $\Delta D$ for a secondary battery being diagnosed using a fifth trained model adapted to estimate the difference $\Delta D$ between an electrolyte diffusion coefficient of the secondary battery and a threshold value of electrolyte diffusion coefficient, that is the value at which the secondary battery becomes unsuitable for reuse, based on input data containing items (1) to (8) provided below:

(1) a rated capacity (initial capacity) of the secondary battery;
(2) a discharge capacity measured at a discharge rate equivalent to that for a measurement of the rated capacity; and
(3) a magnitude of electric current for measurements of the following items, (4) to (6):
(4) a voltage drop as determined at a predetermined period of time from initiation of discharge (or a voltage rise as determined at a predetermined period of time from initiation of charge);
(5) a voltage drop as determined at a predetermined SOC;
(6) a temperature rise as determined at a predetermined SOC;
(7) an electrode area; and
(8) a positive-electrode application quantity.

EFFECTS OF THE INVENTION

The present invention enables estimating, in a simple manner, the electrolyte diffusion coefficient of a secondary battery without disassembly. Further, the present invention enables evaluating the remaining life of a secondary battery more accurately than conventional techniques.

BRIEF DESCRIPTION OF THE DRAWINGS

[FIG. 1] FIG. 1 is a flow chart of a method of diagnosing a secondary battery according to one embodiment of the present invention.
[FIG. 2] FIG. 2 is a flow chart illustrating a more specific exemplary procedure of the step of estimating characteristic parameters.
[FIG. 3] FIG. 3 is a graph illustrating an exemplary relationship between electrolyte diffusion coefficient and discharge capacity.
[FIG. 4] FIG. 4 illustrates an exemplary manner in which a threshold value Dth is determined.
[FIG. 5] FIG. 5 is a flow chart of a method of diagnosing a secondary battery according to a first embodiment of the present invention.
[FIG. 6] FIG. 6 is a flowchart of a method of diagnosing a secondary battery according to a second embodiment of the present invention.
[FIG. 7] FIG. 7 is a flow chart of a method of diagnosing a secondary battery according to a third embodiment of the present invention.
[FIG. 8] FIG. 8 is a flow chart of a method of diagnosing a secondary battery according to a fourth embodiment of the present invention.
[FIG. 9] FIG. 9 shows the relationship between the difference $\Delta D$ obtained by diagnosis and the number of cycles at the onset of a sharp drop.

[FIG. 10] FIG. 10 illustrates a procedure of machine learning.

[FIG. 11] FIG. 11 is a scatter diagram obtained by plotting predicted threshold values of electrolyte diffusion coefficient on the vertical axis and true values (values obtained by analysis) on the horizontal axis with respect to training data.

[FIG. 12] FIG. 12 is a scatter diagram obtained by plotting predicted threshold values of electrolyte diffusion coefficient on the vertical axis and true values (values obtained by analysis) on the horizontal axis with respect to validation data.

[FIG. 13] FIG. 13 is a scatter diagram obtained by plotting predicted threshold values of electrolyte diffusion coefficient on the vertical axis and true values (values obtained by analysis) on the horizontal axis with respect to training data.

[FIG. 14] FIG. 14 is a scatter diagram obtained by plotting predicted threshold values of electrolyte diffusion coefficient on the vertical axis and true values (values obtained by analysis) on the horizontal axis with respect to validation data.

[FIG. 15] FIG. 15 is a scatter diagram obtained by plotting predicted values of electrolyte conductivity on the vertical axis and true values (values obtained by analysis) on the horizontal axis with respect to training data.

[FIG. 16] FIG. 16 is a scatter diagram obtained by plotting predicted values of electrolyte conductivity on the vertical axis and true values (values obtained by analysis) on the horizontal axis with respect to validation data.

[FIG. 17] FIG. 17 is a scatter diagram obtained by plotting predicted values of electrolyte diffusion coefficient on the vertical axis and true values (values obtained by analysis) on the horizontal axis with respect to training data.

[FIG. 18] FIG. 18 is a scatter diagram obtained by plotting predicted values of electrolyte diffusion coefficient on the vertical axis and true values (values obtained by analysis) on the horizontal axis with respect to validation data.

## EMBODIMENTS FOR CARRYING OUT THE INVENTION

In the past, the present inventors developed a method of diagnosing a secondary battery focusing on electrolyte diffusion coefficient (PCT/JP2023/003516, WO 2023/149532). According to this diagnostic method, the load characteristics of a secondary battery are measured and, based on the data obtained and a predetermined model equation, characteristic parameters of the secondary battery being diagnosed at the time of diagnosis (e.g., electrolyte diffusion coefficient $D_n$ and electrolyte conductivity) are estimated. Next, based on a model equation and the estimated parameters, the relationship between electrolyte diffusion coefficient and discharge capacity is determined. Based on this relationship between electrolyte diffusion coefficient and discharge capacity, the value of electrolyte diffusion coefficient at which the secondary battery becomes not suitable for reuse (i.e., threshold value), $D_{th}$, is determined. The larger the difference between the electrolyte diffusion coefficient $D_n$ at the time of diagnosis and the threshold value $D_{th}$, $\Delta D = D_n - D_{th}$, the higher the evaluated possibility that the secondary battery being diagnosed can still be used for a long time (i.e., the remaining life is long).

In this method of diagnosing a secondary battery, to determine a threshold value $D_{th}$, a simulation must be performed for each secondary battery subject to determination to obtain the relationship between electrolyte diffusion coefficient and discharge capacity. This simulation requires a certain time and also requires one to be skilled in utilizing the software used for the simulation. In view of this, to allow one to diagnose a secondary battery in a simpler manner, it will be preferable if a threshold value $D_{th}$ can be determined without such a simulation.

The present inventors did research on this issue, too, and developed a method of estimating the threshold value $D_{th}$ using an estimation model generated through machine learning (PCT/JP2023/027999, WO 2024/029501). Specifically, first, for a plurality of secondary batteries with, for example, different types, different use conditions and different degrees of deterioration of positive and negative-electrode active materials, the above-described simulation was conducted to determine a threshold value $D_{th}$. Next, they built an estimation model (trained model) through machine learning, where some of the characteristic parameters of the secondary batteries represented the input data (explanatory variables) and the determined threshold value $D_{th}$ represented the output data (target variables), and, using this model, successfully estimated the threshold value $D_{th}$ for a secondary battery with an unknown threshold value $D_{th}$. During this, they discovered that accuracy in estimation can be further enhanced by including the discharge capacity and electrolyte conductivity of the secondary battery in the input data.

This method of diagnosing a secondary battery using a trained model allows omitting a simulation for deciding on a threshold value $D_{th}$. On the other hand, a simulation needs to be performed to estimate the electrolyte diffusion coefficient $D_n$ at the time of diagnosis, and at least one round of simulation needs to be performed to calculate $\Delta D = D_n - D_{th}$.

The inventors continued their research and succeeded in building a trained model that estimates the electrolyte diffusion coefficient $D_n$ at the time of diagnosis from characteristic parameters that can be obtained without performing a simulation (specifically, battery specification data and data that can be obtained by simple measurement). Similarly, as for electrolyte conductivity, they succeeded in building a trained model that estimates the electrolyte conductivity at the time of diagnosis from characteristic parameters that can be obtained without performing a simulation. This made all simulation-based analysis unnecessary and enabled obtaining $\Delta D = D_n - D_{th}$ only based on data that can be obtained from battery specification data and simple measurement.

The present invention was made based on these findings. Now, embodiments of the present invention will be described in detail with reference to the drawings.

[Method of Diagnosing Secondary Battery]

In the following description, first, a method of determining $\Delta D$ without the use of a machine learning-based estimation model (hereinafter referred to as "trained model") will be described. Then, a method of determining $\Delta D$ using a trained model will be described.

[Method of Determining $\Delta D$ without Use of Trained Model]

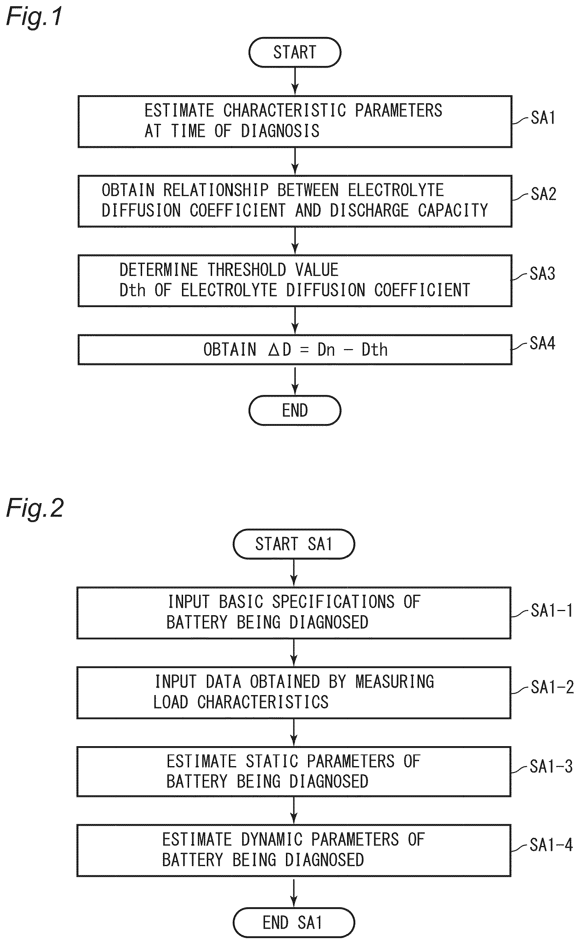

FIG. 1 is a flowchart of a method of diagnosing a secondary battery according to one embodiment of the present invention (i.e., an implementation where no trained model is used). This diagnostic method includes the step of estimating characteristic parameters of a secondary battery to be diagnosed (hereinafter "battery being diagnosed") at a time of diagnosis (step SA1), the step of obtaining the relationship between the electrolyte diffusion coefficient and the discharge capacity (step SA2), the step of determining the threshold value Dth of the electrolyte diffusion coefficient (step SA3), and the step of obtaining the difference $\Delta D$ between the threshold value Dth and the electrolyte diffusion coefficient Dn at the time of diagnosis (step SA4). Now, each step will be described in detail.

[Step of Estimating Characteristic Parameters]

Characteristic parameters of the battery being diagnosed at the time of diagnosis are estimated (step SA1). More specifically, on the basis of the data obtained by measuring the load characteristics of the battery being diagnosed, the characteristic parameters of the battery being diagnosed at the time of diagnosis, including the electrolyte diffusion coefficient Dn of the battery being diagnosed at the time of diagnosis, are estimated using a predetermined model equation.

In this step, characteristic parameters of the battery being diagnosed at the time of diagnosis are estimated by fitting the data obtained by measuring the load characteristics of the battery being diagnosed using a predetermined model equation. This analysis (simulation) can be performed by a computer program that can perform fluid analysis, and can be performed by, for example, the software Battery Design Studio available from Siemens AG.

The model equation used can be an equation well known in this field. As the model equation, for example, the equation described in Marc Doyle et al., "Modeling of Galvanostatic Charge and Discharge of the Lithium/Polymer/Insertion Cell," J. Electrochem. Soc., Vol. 140, No. 6, June (1993), can be used.

The battery being diagnosed is, for example, a lithium ion battery.

The data obtained by measuring the load characteristics of the battery being diagnosed is, for example, a discharge curve obtained by measuring the battery being diagnosed at a plurality of discharge rates. This data preferably includes a discharge curve obtained by measurement at an extremely low discharge rate (0.02 C, for example). Further, this data preferably includes a discharge curve obtained by measurement at a discharge rate of 1 C or higher. This data preferably includes a discharge curve obtained by measurement at three or more discharge rate levels, and more preferably includes a discharge curve obtained by measurement at four or more discharge rate levels. The data obtained by measuring the load characteristics of the battery being diagnosed may be a charge curve obtained by measuring the battery being diagnosed at a plurality of charge rates.

The characteristic parameters estimated in this step (characteristic parameters of the battery being diagnosed at the time of diagnosis) include at least the electrolyte diffusion coefficient Dn of the battery being diagnosed at the time of diagnosis. The characteristic parameters can include, for example, a solid-phase diffusion coefficient of positive and negative-electrode active materials and an electrolyte conductivity. Other specific examples of characteristic parameters will be described below.

FIG. 2 is a flowchart illustrating an example of a more specific procedure of the step of estimating characteristic parameters (step SA1). In this example, the step of estimating the characteristic parameters (step SA1) includes the step of inputting basic specifications of the battery being diagnosed (step SA1-1), the step of inputting data obtained by measuring load characteristics of the battery being diagnosed (step SA1-2), the step of estimating static parameters of the battery being diagnosed (step SA1-3), and the step of estimating dynamic parameters of the battery being diagnosed (step SA1-4).

Basic specifications of the secondary battery to be diagnosed are input to the analysis software (step SA1-1). The basic specifications input can include, but are not limited to, for example, the following:

- Electrode compositions of positive and negative electrodes (constituent materials, content ratios, particle sizes, and the like)
- Electrode thicknesses, densities, and tortuosities (approximately 1.5 in many cases) of positive and negative electrodes

- Materials, thicknesses, and electrical conductivities of positive and negative electrode current-collecting foils
- Thickness and porosity of separator
- Composition of electrolyte (constituent materials, content ratios)
- Thermal conductivities and heat capacities of the constituent materials (basic physical property values specific to the materials)
- Electrode area

With the diagnosis being basically performed non-destructively, accurate composition information of the electrolyte at the time of diagnosis cannot be obtained. In view of this, general information of the secondary battery to be diagnosed (or standard information of a new battery) is obtained and input as parameters. Although some values need to be input when the simulation is actually performed, the composition of the electrolyte does not significantly affect the result of the simulation. In the diagnostic method of the present embodiment, the role of the electrolyte composition information is only for reference purposes.

Although the densities of the positive and negative electrodes are also expected to change due to expansion from initial states, accurate values at the time of diagnosis cannot be measured. In view of this, an initial value (standard value or the like) or a value predicted from the initial value is input. If the value is completely unknown, a typical value may be input. If necessary, fine adjustment may be performed in step SA1-4.

The data obtained by measuring the load characteristics of the battery being diagnosed is input to the analysis software (step SA1-2). The data obtained by measuring the load characteristics of the battery being diagnosed is, as described above, a discharge curve obtained by measuring the battery being diagnosed at a plurality of discharge rates, or the like. The "data obtained by measuring the load characteristics of the battery being diagnosed" will hereinafter also be referred to as "actual measurement data".

The static parameters of the battery being diagnosed are estimated based on the actual measurement data and the model equation (step SA1-3). For example, the static parameters of the battery being diagnosed are adjusted so as to match a shape of the discharge curve obtained by measurement at an extremely low discharge rate. The discharge curve obtained by measurement at an extremely low discharge rate (0.02 C, for example) can be considered to approximately match a voltage curve found when no load is connected (open-circuit voltage curve (OCV)). The static parameters can include, but are not limited to, for example, the following.

- Capacities per unit weight of positive and negative-electrode active materials (battery after use decreases in discharge capacity)
- Respective utilization rates of positive and negative-electrode active materials (not all regions are mutually used)
- Maximum voltage and minimum voltage of battery being diagnosed usage range

The dynamic parameters of the battery being diagnosed are estimated based on the actual measurement data and the model equation (step SA1-4). For example, a simulation is performed in which the battery being diagnosed is discharged at a current value equivalent to that of the measurement conditions of actual measurement data, and the dynamic parameters are adjusted while comparing the data obtained by this simulation result and the actual measurement data such that the two match. This simulation can be performed using, for example, the discharge curve prediction of Battery Design Studio mentioned above. The dynamic parameters may include, but are not limited to, for example, the following.

- Electrolyte conductivity
- Electrolyte diffusion coefficient
- Solid phase diffusion coefficients of positive and negative-electrode active materials
- Thermal capacity of battery being diagnosed

The ambient temperature at the time of the simulation is preferably set so as to match the ambient temperature at the time of actual measurement data acquisition. In the case of a medium or larger sized product battery, particularly a product battery assumed to be used at a high rate, preferably the influence of heat generation is taken into consideration. To achieve this, preferably, measurements are performed at least at 1 C and fitting is conducted with the actual measurement data affected by heat generation. On the other hand, if the battery being diagnosed is a small cell for a desk test or the like, the influence of heat generation need not be considered.

The above steps make it possible to estimate the characteristic parameters of the battery being diagnosed at the time of diagnosis, including the electrolyte diffusion coefficient $D_n$ of the battery being diagnosed at the time of diagnosis.

[Step of Obtaining Relationship between Electrolyte Diffusion Coefficient and Discharge Capacity]

On the basis of the model equation used in step S1 and the characteristic parameters estimated in step SA1, the relationship between the electrolyte diffusion coefficient and the discharge capacity is obtained (step SA2). More specifically, the discharge capacity is obtained by performing a discharge simulation while changing, among the characteristic parameters estimated in step SA1, only the electrolyte diffusion coefficient and keeping the other characteristic parameters constant. A discharge rate and an ambient temperature at the time of the discharge simulation are preferably set in accordance with the reuse application. For example, if the battery being diagnosed is expected to be used in an application at an average rate of about 1 C, then the discharge rate used when obtaining the relationship between the electrolyte diffusion coefficient and the discharge capacity is also 1 C. Since it is difficult to precisely match all environmental conditions, simulations may be performed using average values.

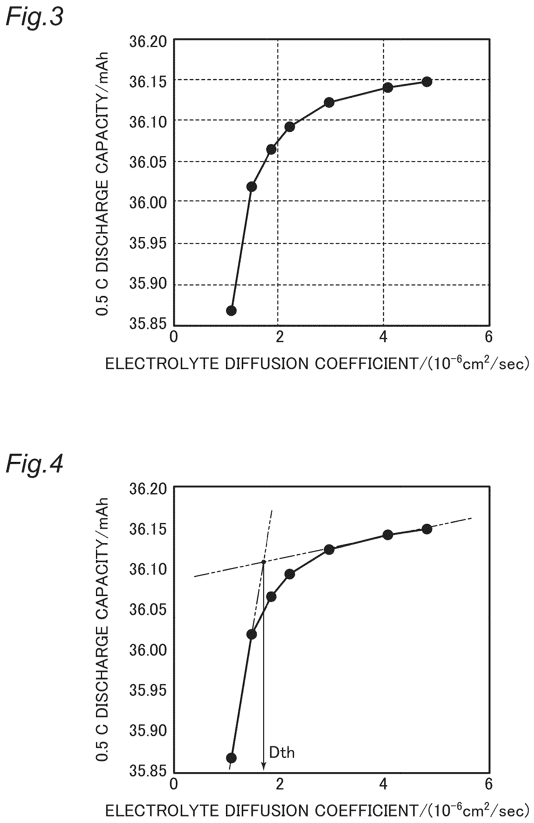

FIG. 3 is a graph showing an example of a relationship between the electrolyte diffusion coefficient and the discharge capacity. In this example, the discharge capacity with the ambient temperature set to 45 °C and the discharge rate set to 0.5 C was obtained for each of the cases in which the electrolyte diffusion coefficient was $4.8 \times 10^{-6}$, $4.0 \times 10^{-6}$, $2.95 \times 10^{-6}$, $1.85 \times 10^{-6}$, $1.48 \times 10^{-6}$, and $1.1 \times 10^{-6}$ cm$^2$/s.

As shown in this example, generally, the smaller the electrolyte diffusion coefficient, the smaller the discharge capacity. Further, the relationship between the electrolyte diffusion coefficient and the discharge capacity is not linear, but rather has a tendency to exhibit a curve such that the decrease in discharge capacity increases as the electrolyte diffusion coefficient decreases.

[Step of Determining Threshold Value Dth of Electrolyte Diffusion Coefficient]

On the basis of the relationship between the electrolyte diffusion coefficient and the discharge capacity obtained in step SA2, the threshold value Dth of the electrolyte diffusion coefficient is determined (step SA3). More specifically, with reference to the relationship between the electrolyte diffusion coefficient and the discharge capacity obtained in step SA2, the value of the electrolyte diffusion coefficient when the battery being diagnosed becomes unsuitable for reuse is determined to be the threshold value Dth. A criterion for determining that the battery being diagnosed is "not suitable for reuse" differs according to the intended reuse application of the battery being diagnosed. As such, the criterion for determining that the battery being diagnosed is "not suitable for reuse" is set in accordance with the application.

For example, when the discharge capacity becomes equal to or less than a predetermined permissible value, the determination may be made that the battery is not suitable for reuse. In this case, it is decided that the electrolyte diffusion coefficient when the discharge capacity becomes equal to or less than the predetermined permissible value is treated as threshold value Dth. For example, in the example of FIG. 3, in a case in which the permissible value of the discharge capacity is 36.02 mAh, the threshold value Dth is $1.40 \times 10^{-6}$ cm$^2$/s.

Alternatively, at the onset of a sharp drop in discharge capacity, it may be determined that the battery is not suitable for reuse. In this case, it is decided that the electrolyte diffusion coefficient at the onset of a sharp drop in discharge capacity is treated as the threshold value Dth. For example, the electrolyte diffusion coefficient when a slope of the discharge capacity is equal to or greater than a predetermined magnitude may be set as the threshold value Dth. Further, as shown in FIG. 4, a point at which tangents of each curve before and after the onset of a sharp drop in discharge capacity intersect may be set as the threshold value Dth.

[Step of Obtaining Difference ΔD between Threshold Value Dth and Electrolyte Diffusion Coefficient Dn at Time of Diagnosis]

A difference ΔD between the threshold value Dth determined in step SA3 and the electrolyte diffusion coefficient Dn at the time of diagnosis estimated in step SA1 is obtained (step SA4). For example, if Dn = $2.22 \times 10^{-6}$ cm$^2$/s and Dth = $1.40 \times 10^{-6}$ cm$^2$/s, then, ΔD = Dn - Dth = $0.82 \times 10^{-6}$ cm$^2$/s.

This difference ΔD can be used as an indicator of the remaining life of the battery being diagnosed. That is, the battery being diagnosed can be evaluated as follows: the larger the value of ΔD, the higher the likelihood the battery being diagnosed can be used for a long period of time, and the smaller the value of ΔD, the lower the likelihood the battery being diagnosed can be used for a long period of time. Even if the discharge capacities at the time of diagnosis are about the same, ΔD may be different. The use of ΔD makes it possible to more accurately evaluate the remaining life of the battery being diagnosed as compared with known methods in which the remaining life is evaluated on the basis of the magnitude of the discharge capacity at the time of diagnosis.

It will be understood that the life of a secondary battery greatly varies depending on usage conditions, and thus "accurate evaluation of the remaining life" does not necessarily mean prediction of a specific number of charge/discharge cycles until unsuitable for reuse. However, if it is assumed that the battery being diagnosed is continuously used under certain conditions, it is also possible to predict the remaining life (number of charge/discharge cycles) of the battery being

diagnosed from ∆D. For example, a relationship between ∆D and the remaining life may be measured in advance, and the remaining life of the battery being diagnosed may be predicted on the basis of this relationship between ∆D and the remaining life.

[Method of Determining ∆D Using Trained Model]

[First Embodiment]

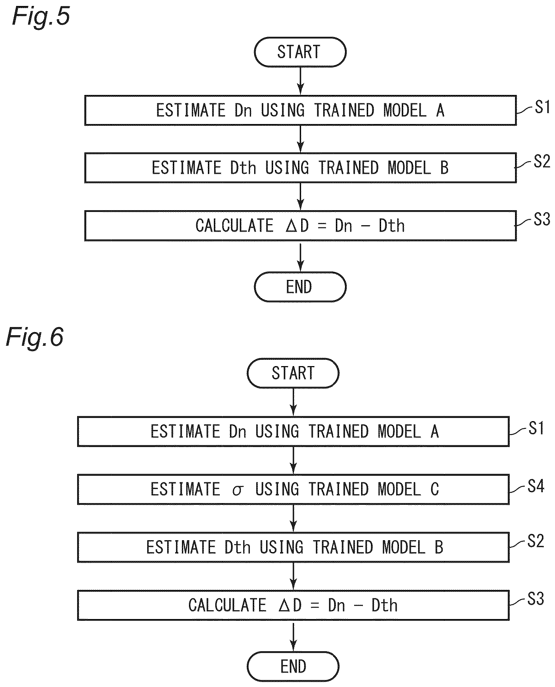

FIG. 5 is a flow chart of a method of diagnosing a secondary battery according to a first embodiment of the present invention. This diagnostic method includes the step of estimating an electrolyte diffusion coefficient at a time of diagnosis of a battery being diagnosed, Dn, using a first trained model (hereinafter referred to as "trained model A") (step S1), the step of estimating a threshold value of electrolyte diffusion coefficient of the battery being diagnosed, Dth, using a second trained model (hereinafter referred to as "trained model B") (step S2), and the step of calculating the difference between the threshold value Dth and the electrolyte diffusion coefficient Dn at the time of diagnosis, ∆D (step S3). The order of steps S1 and S2 may be reversed.

[Step of Estimating Electrolyte Diffusion Coefficient Dn Using Trained Model A]

An electrolyte diffusion coefficient Dn at a time of diagnosis of a battery being diagnosed is estimated using the trained model A (step S1). The trained model A is an estimation model that estimates the electrolyte diffusion coefficient Dn based on predetermined input data.

Specifically, the input data for the trained model A preferably contains: (1) the rated capacity (i.e., initial capacity) of the secondary battery; (2) the discharge capacity measured at a discharge rate equivalent to that for the measurement of the rated capacity; (3) the magnitude of electric current; (4) the voltage drop as determined at a predetermined period of time from initiation of discharge (or the voltage rise as determined at a predetermined period of time from initiation of charge); (5) the voltage drop as determined at a predetermined SOC; and (6) the temperature rise as determined at a predetermined SOC.

The rated capacity of item (1) above is the capacity of the secondary battery before deterioration, i.e., its initial capacity. Typically, the rated capacity of a secondary battery is measured at a relatively low discharge rate, such as 0.2 C. The discharge capacity of item (2) above is the capacity of the secondary battery after deterioration, measured at a discharge rate equivalent to that for the measurement of the rated capacity. The discharge rate at the time of the measurement of item (2) is a value of electric current calculated with respect to the initial rated capacity.

The magnitude of electric current of item (3) above is the magnitude of electric current for the measurements of items (4) to (6). Although any magnitude of electric current may be set, it is preferable to set a magnitude of electric current expected during use of the battery. For example, the magnitude of electric current of item (3) above is preferably set to an electric current at 0.5 C to 1.5 C with respect to the initial capacity, and more preferably to a current value at 0.8 C to 1.2 C, which will provide a magnitude of electric current that can be measured in a relatively short period of time and used as a standard.

The voltage drop as determined at a predetermined period of time (or voltage rise as determined at a predetermined period of time from initiation of charge) of item (4) can be obtained from charge data or discharge data (hereinafter referred to as "charge/discharge data"). Although any "predetermined period of time" may be set, shorter periods of time (e.g., not longer than 10 seconds) are preferable so as not to require a long time to obtain such data. Although the charge/discharge data may be replaced by data used for the measurement of item (2) above, it is preferable to use measurements at 0.5 to 1.5 C, as discussed in connection with item (3) above.

The voltage drop as determined at a predetermined SOC of item (5) above can also be obtained from charge/discharge data as discussed above. If the "predetermined SOC" is set to too low a value, measurement may be impossible when the secondary battery has deteriorated. In view of this, the "predetermined SOC" is preferably set to an SOC of 40 % to an SOC of 60 %, and more preferably to an SOC of 45 % to an SOC of 55 %.

The temperature rise as determined at a predetermined SOC of item (6) above may be obtained from the above-described charge/discharge data and the temperature of the secondary battery at the relevant time. It is preferable that the "predetermined SOC" and the SOC set for item (5) above share a common value.

The input data for the trained model A may further contain one or more selected from: (7) the discharge capacity measured at the electric current of item (3) above; (8) the electrode area; (9) the porosity of the positive-electrode mixture layer (or the density of the positive-electrode mixture layer); (10) the porosity of the negative-electrode mixture layer (or the density of the negative-electrode mixture layer); and (11) the thermal capacity of the secondary battery.

The discharge capacity of item (7) above may be obtained from the above-described charge/discharge data. For the electrode area of item (8) above, data about the battery upon production maintained by the manufacturer may be used, for example. For the porosity of the positive-electrode mixture layer of item (9) above and the porosity of the negative-electrode mixture layer of item (10), data about the battery upon production may be used; or, since an approximate typical amount of expansion can be determined from the types of the active materials, it is possible to use the maximum values (or, if density is used, the minimum values) expected after deterioration. The thermal capacity of the secondary battery of item (11) above needs to be calculated through analysis; however, for secondary batteries of the same type, common values before and after deterioration may be used.

In sum, the input data for the trained model A preferably contains items (1) to (6) listed below, and, more preferably, further contains one or more selected from items (7) to (11) listed below to further reduce prediction error. The input data for the trained model A may contain other data (e.g., positive-electrode application quantity, the type of the positive-electrode active material, the thickness of the positive-electrode mixture layer, negative electrode application quantity, the type of the negative-electrode active material, the thickness of the negative-electrode mixture layer, and the thickness of the separator).

(1) The rated capacity (initial capacity) of the secondary battery;
(2) The discharge capacity measured at a discharge rate equivalent to that for the measurement of the rated capacity;
(3) The magnitude of electric current;
(4) The voltage drop as determined at a predetermined period of time from initiation of discharge (or the voltage rise as determined at a predetermined period of time from initiation of charge);
(5) The voltage drop as determined at a predetermined SOC;
(6) The temperature rise as determined at a predetermined SOC;
(7) The discharge capacity measured at the electric current of item (3) above;
(8) The electrode area;
(9) The porosity of the positive-electrode mixture layer (or the density of the positive-electrode mixture layer);
(10) The porosity of the negative-electrode mixture layer (or the density of the negative-electrode mixture layer); and
(11) The thermal capacity of the secondary battery.

The trained model A may be obtained through machine learning using teaching data that has been created in advance for a plurality of secondary batteries (hereinafter referred to as "secondary batteries for learning").

The teaching data is preferably created for a plurality of secondary batteries with different types, different use conditions and different degrees of deterioration of positive/negative-electrode active materials, for example. The larger the number of secondary batteries for learning (i.e., the larger the number of teaching data sets used for machine learning), the higher the accuracy of the trained model that can be obtained.

A step similar to the step of estimating characteristic parameters at a time of diagnosis of the battery being diagnosed (step SA1), described with reference to FIG. 1, is performed on a plurality of secondary batteries for learning, and an electrolyte diffusion coefficient Dn is estimated. Teaching data is created with this electrolyte diffusion coefficient Dn as output data.

The created teaching data is used to generate, through machine learning, an estimation model (trained model) for estimating output data (target variables) based on input data (explanatory variables). The machine learning is not limited to any particular algorithm; for example, nonlinear support vector regression may be used.

[Step of Estimating Threshold Value Dth of Electrolyte Diffusion Coefficient Using Trained Model B]

A threshold value Dth of electrolyte diffusion coefficient is estimated using the trained model B (step S2). The trained model B is an estimation model that estimates the threshold value Dth of electrolyte diffusion coefficient based on predetermined input data.

Specifically, the input data for the trained model B preferably contains: (1) the discharge capacity measured at a discharge rate equivalent to that for the measurement of the rated capacity; (2) the electrolyte conductivity $\sigma$ or the ohmic resistance resulting from conversion to a value for a unit area of the electrode; (3) the electrode area; (4) the magnitude of electric current; and (5) the positive-electrode application quantity. The discharge rate at the time of the measurement of item (1) is a value of electric current calculated with respect to the initial rated capacity.

The "discharge capacity measured at a discharge rate equivalent to that for the measurement of the rated capacity" of item (1) above is analogous to that described in connection with the trained model A.

The electrolyte conductivity $\sigma$ of item (2) above can be calculated by performing a step similar to the step of estimating characteristic parameters at a time of diagnosis of the battery being diagnosed (step SA1), described with reference to FIG. 1. The ohmic resistance resulting from conversion to a value for a unit area of the electrode can be calculated through impedance measurement on the secondary battery. An ohmic resistance is easy to obtain; on the other hand, it also contains, for example, the resistance of the electrode and the contact resistances of parts such as a tab, and may thus provide lower accuracy than in implementations where an electrolyte conductivity $\sigma$ is used.

Items (3) and (4) above are the same as described in connection with the trained model A. The magnitude of electric current of item (3) above may be set to any value; if the magnitude of electric current is used as input data, it and the input data for the trained model A need to share a common value. For the positive-electrode application quantity of item (5) above, data about the battery upon production may be used, for example.

The input data for the trained model B may further contain one or more selected from: (6) the type of the positive-electrode active material; (7) the porosity of the positive-electrode mixture layer (or the density of the positive-electrode mixture layer); (8) the porosity of the negative-electrode mixture layer (or the density of the negative-electrode mixture layer); and (9) the thermal capacity of the secondary battery.

Items (7) to (9) above are the same as described in connection with the trained model A. For the type of the positive-electrode active material of item (6) above, data about the battery upon production may be used, for example.

In sum, the input data for the trained model B preferably contains items (1) to (5) listed below, and, more preferably, further contains one or more selected from items (6) to (9) listed below to further reduce prediction error. The input data for the trained model B may contain other data.

(1) The discharge capacity measured at a discharge rate equivalent to that for the measurement of the rated capacity;
(2) The electrolyte conductivity $\sigma$ or the ohmic resistance resulting from conversion to a value for a unit area of the electrode;
(3) The electrode area;
(4) The magnitude of electric current;
(5) The positive-electrode application quantity;
(6) The type of the positive-electrode active material;
(7) The porosity of the positive-electrode mixture layer (or the density of the positive-electrode mixture layer);
(8) The porosity of the negative-electrode mixture layer (or the density of the negative-electrode mixture layer); and
(9) The thermal capacity of the secondary battery.

Similar to the trained model A, the trained model B is obtained by performing machine learning using teaching data that has been created in advance for a plurality of secondary batteries for learning. Specifically, steps are performed on a plurality of secondary batteries for learning that are similar to the step of estimating characteristic parameters at a time of diagnosis of the battery being diagnosed (step SA1), the step of calculating the relationship between electrolyte diffusion coefficient and discharge capacity (step SA2) and the step of determining the threshold value Dth of electrolyte diffusion coefficient (step SA3), described with reference to FIG. 1, to decide on a threshold value Dth of electrolyte diffusion coefficient. Teaching data is created with this threshold value Dth of electrolyte diffusion coefficient as output data. The created teaching data is used to generate, through machine learning, an estimation model (trained model) for estimating output data (target variables) based on input data (explanatory variables).

[Step of Calculating Difference $\Delta D$ between Threshold Value Dth and Electrolyte Diffusion Coefficient at Time of Diagnosis Dn]

The difference between the threshold value Dth estimated at step S2 and the electrolyte diffusion coefficient at the time of diagnosis Dn estimated at step S1, $\Delta D = Dn - Dth$, is calculated (step S3). This difference $\Delta D$ may serve as an indicator of the remaining life of the battery being diagnosed. The use of the difference $\Delta D$ will enable evaluating the remaining life of the battery being diagnosed more accurately than conventional methods that evaluate the remaining life based on the magnitude of discharge capacity at the time of diagnosis.

The present embodiment will further enable calculating the electrolyte diffusion coefficient Dn at the time of diagnosis of the battery being diagnosed by the use of the trained model A without simulation-based analysis. The present embodiment will also enable calculating the threshold value Dth of electrolyte diffusion coefficient in a simple manner by the use of the trained model B.

[Second Embodiment]

FIG. 6 is a flowchart of a method of diagnosing a secondary battery according to a second embodiment of the present invention. In addition to the steps in the diagnostic method according to the first embodiment (FIG. 5), the present diagnostic method further includes the step of estimating an electrolyte conductivity $\sigma$ at the time of diagnosis of the battery being diagnosed using a third trained model (hereinafter referred to as "trained model C") (step S4).

In the above-discussed trained model B, the input data contains the electrolyte conductivity $\sigma$ or the ohmic resistance resulting from conversion to a value for a unit area of the electrode. The calculation of the electrolyte conductivity $\sigma$ at the time of diagnosis requires simulation-based analysis (step SA1 in FIG. 1). As such, if the electrolyte conductivity $\sigma$ is used as input data, the diagnostic method according to the first embodiment (FIG. 5) cannot allow complete omission of simulation-based analysis during diagnosis. Although it is possible to calculate ohmic resistance without simulation-based analysis, accuracy may then be lower than in implementations where the electrolyte conductivity σ is used.

According to the present embodiment, an electrolyte conductivity σ at a time of diagnosis of the battery being diagnosed is estimated using a trained model C (step S4). This will allow omission of simulation-based analysis during diagnosis while maintaining accuracy of estimation.

The trained model C is an estimation model that estimates the electrolyte conductivity σ based on predetermined input data. Specifically, the input data for the trained model C preferably contains: (1) the discharge capacity measured at a discharge rate equivalent to that for the measurement of the rated capacity; (2) the magnitude of electric current; (3) the voltage drop as determined at a predetermined period of time from initiation of discharge (or the voltage rise as determined at a predetermined period of time from initiation of charge); (4) the electrode area; and (5) the positive-electrode application quantity. These items are the same as described in connection with the trained models A and B. The discharge rate of item (1) above is a value of electric current calculated with respect to the initial rated capacity, and the magnitude of electric current of item (2) above and the input data for the trained model A need to share a common value.

The input data for the trained model C may further contain one or more selected from: (6) the type of the positive-electrode active material; (7) the porosity of the positive-electrode mixture layer (or the density of the positive-electrode mixture layer); (8) the type of the negative-electrode active material; and (9) the porosity of the negative-electrode mixture layer (or the density of the negative-electrode mixture layer).

Items (6) to (7) and (9) above are the same as described in connection with the trained model B. For the type of the negative-electrode active material of item (8) above, data about the battery upon production may be used, for example.

In sum, the input data for the trained model C preferably contains items (1) to (5) listed below, and, more preferably, further contains one or more selected from items (6) to (9) listed below to further reduce prediction error. The input data for the trained model C may contain other data.

(1) The discharge capacity measured at a discharge rate equivalent to that for the measurement of the rated capacity;
(2) The magnitude of electric current;
(3) The voltage drop as determined at a predetermined period of time from initiation of discharge (or the voltage rise as determined at a predetermined period of time from initiation of charge);
(4) The electrode area;
(5) The positive-electrode application quantity;
(6) The type of the positive-electrode active material;
(7) The porosity of the positive-electrode mixture layer (or the density of the positive-electrode mixture layer);
(8) The type of the negative-electrode active material; and
(9) The porosity of the negative-electrode mixture layer (or the density of the negative-electrode active material).

Similar to the trained model A, the trained model C is obtained by performing machine learning using teaching data that has been created in advance for a plurality of secondary batteries for learning. Specifically, a step is performed on a plurality of secondary batteries for learning that is similar to the step of estimating characteristic parameters at a time of diagnosis of the battery being diagnosed (step SA1), described with reference to FIG. 1, to estimate the electrolyte conductivity σ. Teaching data is created with this electrolyte conductivity σ as output data. The created teaching data is used to generate, through machine learning, an estimation model (trained model) for estimating output data (target variables) based on input data (explanatory variables).

[Third Embodiment]

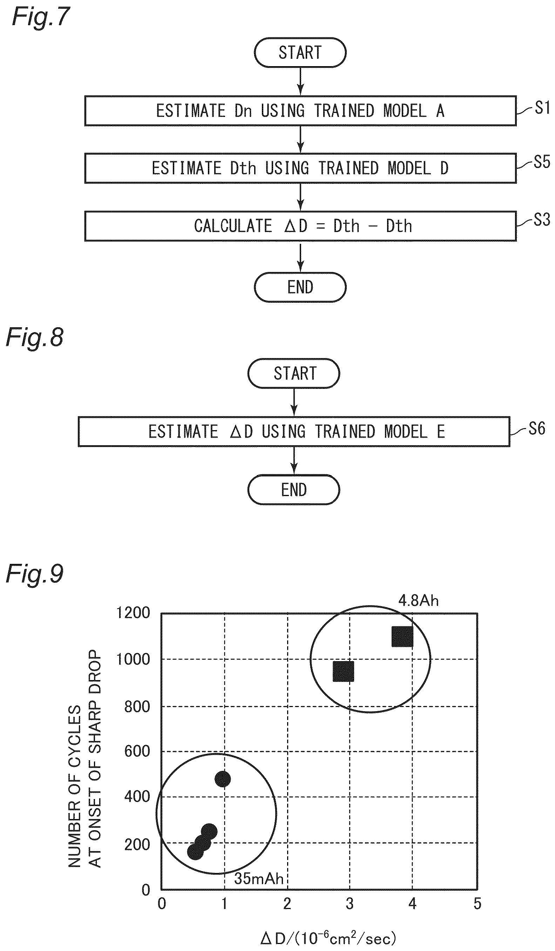

FIG. 7 is a flow chart of a method of diagnosing a secondary battery according to a third embodiment of the present invention. In this diagnostic method, step S2 of the diagnostic method according to the first embodiment (FIG. 5) is replaced by the step of estimating a threshold value Dth of electrolyte diffusion coefficient using a fourth trained model (hereinafter referred to as "trained model D") (step S5).

In the above-discussed diagnostic method according to the second embodiment (FIG. 6), an electrolyte conductivity σ is estimated using the trained model C and, thereafter, a threshold value Dth of electrolyte diffusion coefficient is estimated using the trained model B with that electrolyte conductivity σ as part of the input data. In contrast, in the diagnostic method according to the present embodiment, the step of estimating an electrolyte conductivity σ is not performed, and a threshold value Dth of electrolyte diffusion coefficient is directly estimated based on predetermined input data using the trained model D.

The trained model D is an estimation model that estimates the threshold value Dth of electrolyte diffusion coefficient based on predetermined input data. The input data for the trained model D is a union of the input data for the trained model C and the input data for the trained model B excluding the electrolyte conductivity σ.

In other words, the input data for the trained model D preferably contains items (1) to (5) listed below, and, more preferably, further contains one or more selected from items (6) to (10) listed below to further reduce prediction error. The input data for the trained model D may contain other data.

(1) The discharge capacity measured at a discharge rate equivalent to that for the measurement of the rated capacity;
(2) The magnitude of electric current;
(3) The voltage drop as determined at a predetermined period of time from initiation of discharge (or the voltage rise as determined at a predetermined period of time from initiation of charge);
(4) The electrode area;
(5) The positive-electrode application quantity;
(6) The type of the positive-electrode active material;
(7) The porosity of the positive-electrode mixture layer (or the density of the positive-electrode mixture layer);
(8) The type of the negative-electrode active material;
(9) The porosity of the negative-electrode mixture layer (or the density of the negative-electrode mixture layer); and
(10) The thermal capacity of the secondary battery.

The present embodiment, too, will allow omission of simulation-based analysis during diagnosis while maintaining accuracy of estimation.

[Fourth Embodiment]

FIG. 8 is a flow chart of a method of diagnosing a secondary battery according to a fourth embodiment of the present invention. The present diagnostic method includes the step of estimating $\Delta D = Dn - Dth$ using a fifth trained model (hereinafter referred to as "trained model E").

In the above-discussed diagnostic methods according to the first to third embodiments, an electrolyte diffusion coefficient at a time of diagnosis of the battery being diagnosed $Dn$ and a threshold value $Dth$ of electrolyte diffusion coefficient are estimated, and the difference therebetween, $\Delta D = Dn - Dth$, is calculated. In contrast, in the diagnostic method according to the present embodiment, $\Delta D = Dn - Dth$ is directly estimated based on predetermined input data using the trained model E.

The trained model E is an estimation model that estimates $\Delta D = Dn - Dth$ based on predetermined input data. The input data for the trained model E is a union of the input data for the trained model A and the input data for the trained model D.

In other words, the input data for the trained model E preferably contains items (1) to (8) listed below, and may further contain one or more selected from items (9) to (14) listed below. The input data for the trained model E may contain other data.

(1) The rated capacity (initial capacity) of the secondary battery;
(2) The discharge capacity measured at a discharge rate equivalent to that for the measurement of the rated capacity;
(3) The magnitude of electric current;
(4) The voltage drop as determined at a predetermined period of time from initiation of discharge (or the voltage rise as determined at a predetermined period of time from initiation of charge);
(5) The voltage drop as determined at a predetermined SOC;
(6) The temperature rise as determined at a predetermined SOC;
(7) The electrode area;
(8) The positive-electrode application quantity;
(9) The discharge capacity measured at the electric current of item (3) above;
(10) The type of the positive-electrode active material;
(11) The porosity of the positive-electrode mixture layer (or the density of the positive-electrode mixture layer);
(12) The type of the negative-electrode active material;
(13) The porosity of the negative-electrode mixture layer (or the density of the negative-electrode mixture layer); and
(14) The thermal capacity of the secondary battery.

The present embodiment, too, will allow omission of simulation-based analysis during diagnosis while maintaining accuracy of estimation.

[Program etc. for Diagnosing Secondary Battery]

The above-discussed methods of diagnosing a secondary battery may also be implemented as computer programs. A program for diagnosing a secondary battery according to one embodiment of the present invention causes a computer to perform the step of estimating an electrolyte diffusion coefficient at a time of diagnosis of the secondary battery being diagnosed, Dn, using the trained model A. A program for diagnosing a secondary battery according to another embodiment of the present invention causes a computer to perform the step of estimating the difference $\Delta D$ for the secondary battery being diagnosed using the trained model E. These computer programs may also be ones stored on a computer-readable storage medium.

The above-discussed methods of diagnosing a secondary battery may also be implemented as computer systems. A system for diagnosing a secondary battery according to one embodiment of the present invention includes a memory and a processor, the processor adapted to perform, in accordance with the program on the memory, the step of estimating an electrolyte diffusion coefficient at a time of diagnosis of the secondary battery being diagnosed, Dn, using the trained model A. A system for diagnosing a secondary battery according to another embodiment of the present invention includes a memory and a processor, the processor being adapted to perform, in accordance with the program on the memory, the step of estimating the difference $\Delta D$ for the secondary battery being diagnosed using the trained model E.

An apparatus for diagnosing a secondary battery according to one embodiment of the present invention includes an estimation device adapted to estimate an electrolyte diffusion coefficient at a time of diagnosis of a secondary battery being diagnosed, Dn, using the trained model A. An apparatus for diagnosing a secondary battery according to another embodiment of the present invention includes an estimation device adapted to estimate the difference $\Delta D$ for the secondary battery being diagnosed using the trained model E.

EXAMPLES

Now, the present invention will be described more specifically with reference to examples. The present invention is not limited to these examples.

A plurality of medium-sized laminate-type cells having a rated capacity of 5 Ah and a plurality of small-sized laminate-type cells having a rated capacity of 36 mAh were fabricated.

[Medium-Sized Laminate-Type Cell]

<Fabrication of Positive Electrode>

A slurry containing a positive-electrode mixture was prepared by uniformly mixing 93 parts by mass of $LiCoO_2$ as a positive-electrode active material, 3 parts by mass of carbon black as a conductive aid, and 4 parts by mass of polyvinylidene fluoride (PVDF) as a binder, using N-methyl-2-pyrrolidone (NMP) as a solvent. This slurry containing a positive-electrode mixture was applied to both sides of a positive electrode current collector made of aluminum foil having a thickness of 15 $\mu$m, dried, subsequently pressure-formed using a roller press machine, and then punched out so that a portion of the positive electrode current collector not coated with the slurry containing a positive-electrode mixture formed a tab portion, thereby fabricating the positive electrode.

<Fabrication of Negative Electrode>

A slurry containing a negative-electrode mixture was prepared by mixing 97.5 parts by mass of graphite as a negative-electrode active material, 1.5 parts by mass of carboxymethyl cellulose as a binder, and 1 part by mass of styrene-butadiene rubber, and then adding an appropriate amount of water and thoroughly mixing. This slurry containing a negative-electrode mixture was applied to both sides of a negative electrode current collector made of copper foil having a thickness of 10 $\mu$m, dried, subsequently pressure-formed using a roller press machine, and then punched out so that a portion of the negative electrode current collector not coated with the slurry containing a negative-electrode mixture formed a tab portion, thereby fabricating the negative electrode.

<Fabrication of Battery>

Seven of the positive electrodes described above and eight of the negative electrodes described above were alternately layered with a polyolefin microporous film separator interposed therebetween to form a multilayer electrode body, the polyolefin microporous film separator having a thickness of 18 $\mu$m and a three-layer structure consisting of a polyethylene layer as a middle layer and two polypropylene layers as outer layers.

Next, the tab portions of the positive electrodes of the multilayer electrode body were welded together and the tab portions of the negative electrodes of the multilayer electrode body were welded together before leads were connected to the respective tabs. Then, the multilayer electrode body was enclosed inside an outer packaging made from an aluminum laminate film together with a non-aqueous electrolyte prepared by dissolving $LiPF_6$ at a concentration of 1 mol/L in a solution obtained by mixing ethylene carbonate, diethyl carbonate, and methyl ethyl carbonate at a volume ratio of 1:1:1, and further dissolving vinylene carbonate in an amount of 1 mass%, thereby fabricating a non-aqueous electrolyte secondary battery with a rated capacity of 5 Ah.

[Small-Sized Laminate-Type Cell]

<Fabrication of Positive Electrode>

A slurry containing a positive-electrode mixture was prepared by uniformly mixing 94 parts by mass of $LiCoO_2$ as the positive-electrode active material, 4 parts by mass of carbon black as the conductive aid, and 2 parts by mass of PVDF as the binder, using NMP as the solvent. This slurry containing a positive-electrode mixture was applied to both sides of a positive electrode current collector made of aluminum foil having a thickness of 15 $\mu$m, dried, subsequently pressure-formed using a roller press machine, and then punched out so that a portion of the positive electrode current collector not coated with the slurry containing a positive-electrode mixture formed a tab portion, thereby fabricating the positive electrode.

<Fabrication of Negative Electrode>

A slurry containing a negative-electrode mixture was prepared by mixing 94.5 parts by mass of graphite and 3 parts by mass of SiO particles (D50: 5.0 $\mu$m) including a front surface coated with carbon as the negative-electrode active material, 1.5 parts by mass of carboxymethyl cellulose and 1 part by mass of styrene-butadiene rubber as the binder, and then adding an appropriate amount of water and thoroughly mixing. This slurry containing a negative-electrode mixture was applied to both sides of a negative electrode current collector made of copper foil having a thickness of 10 $\mu$m, dried, subsequently pressure-formed using a roller press machine, and then punched out so that a portion of the negative electrode current collector not coated with the slurry containing a negative-electrode mixture formed a tab portion, thereby fabricating the negative electrode.

<Fabrication of Battery>

The positive electrode described above and the negative electrode described above were layered with a polyolefin microporous film separator interposed therebetween to form a multilayer electrode body, the polyolefin microporous film separator having a thickness of 12 $\mu$m and a three-layer structure consisting of a polyethylene layer as the middle layer and two polypropylene layers as the outer layers.
Next, the tab portions of the positive electrodes of the multilayer electrode body were welded together and the tab portions of the negative electrodes were welded together before leads were connected to the respective tabs. Then, the multilayer electrode body was enclosed inside an outer packaging made from an aluminum laminate film together with a non-aqueous electrolyte prepared by dissolving $LiPF_6$ at a concentration of 1 mol/L in a solution obtained by mixing ethylene carbonate and diethyl carbonate at a volume ratio of 3:7, and further dissolving vinylene carbonate in an amount of 1 mass%, thereby fabricating a non-aqueous electrolyte secondary battery with a rated capacity of 36 mAh.

<Fabrication of Deteriorated Cells>

A plurality of deteriorated cells having discharge capacities reduced to 4.8 Ah were fabricated by conducting charge/discharge cycle tests on the medium-sized laminate-type cells having a rated capacity of 5 Ah under a plurality of conditions different in terms of charge/discharge rate, ambient temperature, and the like. Similarly, a plurality of deteriorated cells having discharge capacities reduced to 35 mAh were fabricated by conducting charge/discharge cycle tests on the small-sized laminate-type cells having a rated capacity of 36 mAh under a plurality of conditions different in charge/discharge rate, ambient temperature, and the like.

[Measurement of Load Characteristics]

The load characteristics of each of the deteriorated cells were measured. Specifically, the discharge curve of each cell was measured at discharge rates of 0.02 C, 0.2 C, 0.5 C and 1 C.

[Diagnosis of Secondary Battery]

The methods of diagnosing a secondary battery described in the embodiments were carried out using these deteriorated cells as batteries being diagnosed. The analysis (simulation) was carried out by the software Battery Design Studio available from Siemens AG. Among the basic specifications, a solvent ratio and a salt concentration were input using the same values as those at the time of fabrication (because the values at the time of diagnosis cannot be measured). **[0109]** The relationship between the electrolyte diffusion coefficient and the discharge capacity was acquired by estimating the characteristic parameters at the time of diagnosis and subsequently, on the basis of the estimated characteristic parameters, obtaining the discharge capacity at an ambient temperature of 45 °C and a discharge rate of 0.5 C while changing the electrolyte diffusion coefficient. The difference ΔD between the threshold value Dth and the electrolyte diffusion coefficient Dn at the time of diagnosis was obtained with the point at which tangents of each curve before and after the onset of a sharp drop in discharge capacity intersect treated as the threshold value Dth.

[Measurement of Remaining Life]

These deteriorated cells were subjected to charge/discharge cycle tests under the same conditions, and the number of charge/discharge cycles until a sharp drop in discharge capacity occurred (number of cycles at the onset of a sharp drop) was measured. FIG. 9 shows the relationship between the difference ΔD obtained by the diagnosis and the number of cycles at the onset of a sharp drop.

As shown in FIG. 9, even when the discharge capacities at the time of diagnosis were equivalent (4.8 Ah or 35 mAh), a difference in ΔD was found. Further, the number of cycles at the onset of a sharp drop was also found to change in accordance with ΔD, confirming the validity of this diagnostic method.

[Generation of Trained model B]

Next, secondary batteries using lithium cobalt oxide (LCO) or lithium nickel cobalt manganese oxide (NCM) as a positive-electrode active material and graphite or SiO as a negative-electrode active material were prepared. The load characteristics of these secondary batteries were measured, and the threshold values of the electrolyte diffusion coefficients were obtained by the method described above. Machine learning was performed using this data as teaching data (however, some data was used not as teaching data (training data) but as verification data (validation data)) to generate a trained model (trained model B).

As input data (explanatory variables), the following nine variables were used:

(1) The discharge capacity measured at a rate equivalent to that for the measurement of the rated capacity (0.2 C);
(2) The electrolyte conductivity $\sigma$;
(3) The electrode area;
(4) The magnitude of electric current;
(5) The positive-electrode application quantity;
(6) The type of positive-electrode active material;
(7) The porosity of the positive-electrode mixture layer;
(8) The porosity of the negative-electrode mixture layer; and
(9) The thermal capacity of the secondary battery.

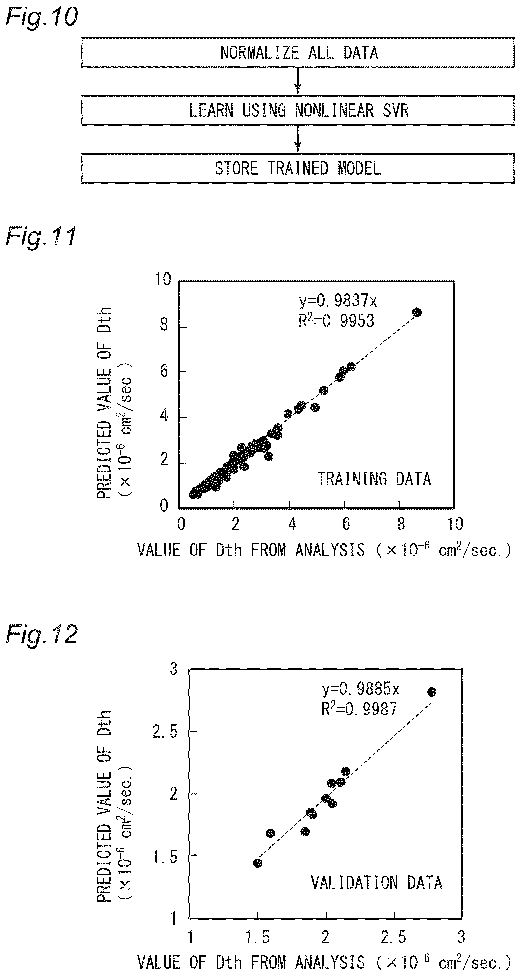

The machine learning was performed using scikit-learn, an open source library. FIG. 10 illustrates the procedure of the machine learning. The algorithm used for machine learning was nonlinear support vector regression (nonlinear SVR).

First, all data were normalized by the following equation:

$$\mathrm{Xn} = (\mathrm{X} - \mathrm{Xa})/\mathrm{Xd}$$

X: original value, Xn: value after normalization, Xa: average value of variable X, Xd: standard deviation

In the non-linear SVR, a weighting coefficient w that would mimimize the following function was obtained:

[Math. Exp. 1]

$$\frac{1}{2}\|\mathbf{w}\|^2 \quad + \quad C\sum_{i=1}^{n} h\left(y^{(i)} - f\left(\mathbf{x}^{(i)}\right)\right)$$

$$f\left(\mathbf{x}^{(i)}\right) = \phi\left(\mathbf{x}^{(i)}\right)\mathbf{w} + c$$

$\Phi(x)$: parameter after nonlinear mapping of original x, w: weighting coefficient of each parameter, c: constant, y: target variable (threshold value of electrolyte diffusion coefficient)

$h(y^{(i)} - f(x^{(i)}))$ is a function represented by the following equation:

$$h(y^{(i)} - f(x^{(i)})) = \max(0, \mid y^{(i)} - f(x^{(i)}) \mid - \varepsilon)$$

C and $\varepsilon$ are so-called hyperparameters.

FIG. 11 and FIG. 12 are scatter diagrams obtained by plotting predicted threshold values of the electrolyte diffusion coefficient on the vertical axis and true values (values obtained by analysis) on the horizontal axis. FIG. 11 is a diagram relating to training data (100 data used) and FIG. 12 is a diagram relating to validation data (data not used for learning). As shown in FIG. 12, the values obtained by analysis were predicted with good accuracy even for data that had not been learned.

<Contribution of Electrolyte Conductivity to Dth>

The influence of electrolyte conductivity on Dth was studied using a trained model.

For a certain secondary battery, when the electrolyte conductivity was decreased from 8.90 (mS/cm) to 1.75 (mS/cm) without changing other parameters, Dth increased from $2.705 \times 10^{-6}$ (cm$^2$/s) to $2.758 \times 10^{-6}$ (cm$^2$/s).

For the same secondary battery, when the thermal capacity was halved without changing the electrolyte conductivity, Dth decreased from $2.705 \times 10^{-6}$ (cm$^2$/s) to $2.643 \times 10^{-6}$ (cm$^2$/s).

Next, a case in which the thermal capacity is halved and the electrolyte conductivity is reduced to 1.75 (mS/cm) will be considered. From the results described above, it is expected that Dth will be $2.643 \times 10^{-6}$ (cm$^2$/s) by halving the thermal capacity, and then will increase by reducing the electrolyte conductivity. However, according to the results of the trained model, the Dth decreased from $2.643 \times 10^{-6}$ (cm$^2$/s) to $2.575 \times 10^{-6}$ (cm$^2$/s).

Thus, depending on the amount of change in other parameters, the influence of the electrolyte conductivity on Dth is not constant, and a correlation between parameters exists. This reveals that simple prediction is difficult.

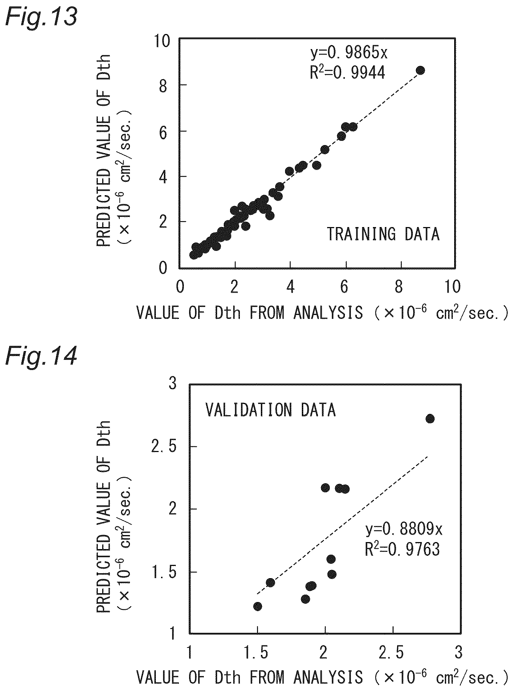

For comparison, machine learning was performed by removing the electrolyte conductivity from the input data (explanatory variables), and a trained model was created. FIG. 13 and FIG. 14 are scatter diagrams obtained by plotting predicted threshold values of the electrolyte diffusion coefficient on the vertical axis and true values (values obtained by analysis) on the horizontal axis. FIG. 13 is a diagram relating to training data, and FIG. 14 is a diagram relating to validation data (data not used for learning). As shown in FIG. 14, in this trained model, the accuracy of prediction was low as compared with the result of the trained model in which the electrolyte conductivity was included in the input data (FIG. 12).

This shows that the accuracy of prediction can be improved by including the electrolyte conductivity in the input data.

[Estimation of Electrolyte Conductivity]

Secondary batteries with a positive-electrode active material of LCO or NCM and a negative-electrode active material of graphite or SiO were prepared. The load characteristics of these secondary batteries were measured and the electrolyte conductivity was estimated through a simulation-based analysis. These datasets were used as teaching data to perform machine learning similar to that for generating a trained model for estimating the threshold value Dth, thus generating a trained model for estimating electrolyte conductivity (i.e., trained model C). As input data (explanatory variables), the following nine variables were used:

(1) the discharge capacity measured at a discharge rate equivalent to that for the measurement of the rated capacity (0.2 C);
(2) the magnitude of electric current;
(3) the voltage drop for one second from initiation of discharge;
(4) the electrode area;
(5) the positive-electrode application quantity;
(6) the type of the positive-electrode active material;
(7) the porosity of the positive-electrode mixture layer;
(8) the type of the negative-electrode active material; and
(9) the porosity of the negative-electrode mixture layer.

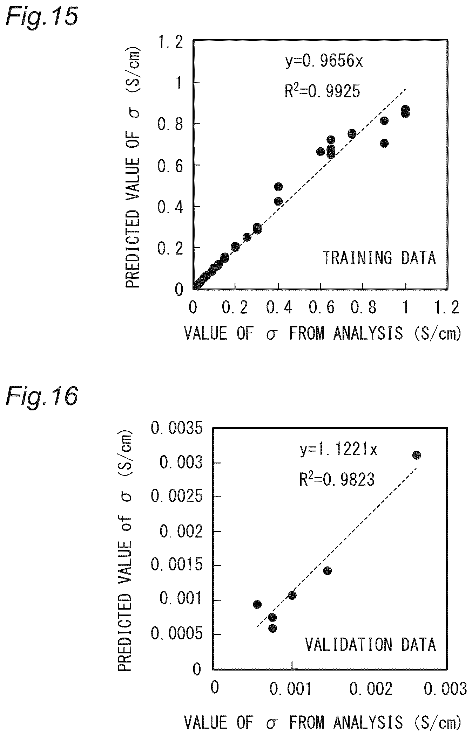

Each of FIGS. 15 and 16 is a scatter diagram obtained by plotting predicted values of electrolyte conductivity $\sigma$ on the vertical axis and plotting true values (values obtained by analysis) on the horizontal axis. FIG. 15 relates to training data, while FIG. 16 relates to validation data. As shown in FIG. 16, the values obtained by analysis were predicted fairly precisely even for data that had not be learned. It is expected that further accumulating training data will improve precision.

[Estimation of Electrolyte Diffusion Coefficient]

Secondary batteries were prepared that included LCO or NCM serving as positive-electrode active material and graphite or SiO serving as negative-electrode active material. The load characteristics of these secondary batteries were measured, and simulation-based analysis was conducted to estimate the electrolyte diffusion coefficient. These datasets were used as teaching data to perform machine learning similar to that for generating the trained model for estimating the threshold value Dth, thus generating a trained model for estimating the electrolyte diffusion coefficient (i.e., trained model A). As input data (explanatory variables), the following eleven variables were used:

(1) the rated capacity (initial capacity) of the secondary battery;
(2) the discharge capacity measured at a discharge rate equivalent to that for the measurement of the rated capacity (0.2 C);
(3) the magnitude of electric current (1 C);
(4) the voltage drop for one second from initiation of discharge;
(5) the voltage drop as determined at SOC 50 %;
(6) the temperature rise as determined at SOC 50 %;
(7) the discharge capacity measured at the electric current of item (3) above;
(8) the electrode area;
(9) the density of the positive-electrode mixture layer;
(10) the density of the negative-electrode mixture layer; and
(11) the thermal capacity of the secondary battery.

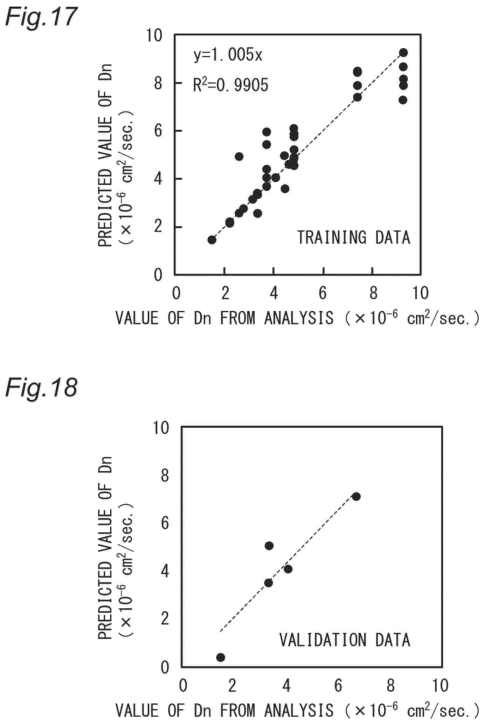

Each of FIGS. 17 and 18 is a scatter diagram obtained by plotting predicted values of the electrolyte diffusion coefficient Dn on the vertical axis and true values (values obtained by analysis) on the horizontal axis. FIG. 17 relates to training data, while FIG. 18 relates to validation data. As shown in FIG. 18, the values obtained by analysis were predicted fairly precisely even for data that had not be learned. It is expected that further accumulating training data will improve precision.

Although embodiments of the present invention have been described, the present invention is not limited to the above-described embodiments, and various modifications can be made within the scope of the invention. For example, the above-described embodiments show implementations where the secondary battery is a single cell; in implementations where the secondary battery is an assembled cell, not only may the present invention be applied to each individual cell, but may also be applied to the entire assembled cell treated as one cell.

**Claims**

1. A method of diagnosing a secondary battery comprising:
   estimating an electrolyte diffusion coefficient at a time of diagnosis of a secondary battery being diagnosed, Dn, using a first trained model adapted to estimate the electrolyte diffusion coefficient of the secondary battery based on input data containing the following items, (1) to (6):

   (1) a rated capacity (initial capacity) of the secondary battery;
   (2) a discharge capacity measured at a discharge rate equivalent to that for a measurement of the rated capacity; and
   (3) a magnitude of electric current for measurements of the following items, (4) to (6):
   (4) a voltage drop as determined at a predetermined period of time from initiation of discharge (or a voltage rise as determined at a predetermined period of time from initiation of charge);
   (5) a voltage drop as determined at a predetermined SOC; and
   (6) a temperature rise as determined at a predetermined SOC.

2. The method of diagnosing a secondary battery according to claim 1, wherein the input data for the first trained model further contains one or more selected from the following items, (7) to (11):

   (7) a discharge capacity measured at the electric current of item (3) above;
   (8) an electrode area;

(9) a porosity of a positive-electrode mixture layer (or a density of a positive-electrode mixture layer);
(10) a porosity of a negative-electrode mixture layer (or a density of a negative-electrode mixture layer); and
(11) a thermal capacity of the secondary battery.

3. The method of diagnosing a secondary battery according to claim 1 or 2, further comprising:

estimating a threshold value of electrolyte diffusion coefficient of the secondary battery being diagnosed, Dth, using a second trained model adapted to estimate the threshold value of electrolyte diffusion coefficient, that is the value at which the secondary battery becomes unsuitable for reuse, based on predetermined input data; and calculating a difference between the threshold value Dth and the electrolyte diffusion coefficient Dn at the time of diagnosis, $\Delta D$.

4. The method of diagnosing a secondary battery according to claim 3, wherein the input data for the second trained model contains the following items, (1) to (5):

(1) a discharge capacity measured at a discharge rate equivalent to that for the measurement of the rated capacity;
(2) an electrolyte conductivity $\sigma$ or an ohmic resistance resulting from conversion to a value for a unit area of the electrode;
(3) an electrode area;
(4) a magnitude of electric current equal to that of item (3) of the input data for the first trained model; and
(5) a positive-electrode application quantity.

5. The method of diagnosing a secondary battery according to claim 4, further comprising:
estimating an electrolyte conductivity $\sigma$ of the secondary battery being diagnosed using a third trained model adapted to estimate the electrolyte conductivity of the secondary battery based on predetermined input data.

6. The method of diagnosing a secondary battery according to claim 5, wherein the input data for the third trained model contains the following items, (1) to (5):

(1) a discharge capacity measured at a discharge rate equivalent to that for the measurement of the rated capacity;
(2) a magnitude of electric current equal to that of item (3) of the input data for the first trained model;
(3) a voltage drop as determined at a predetermined period of time from initiation of discharge (or a voltage rise as determined at a predetermined period of time from initiation of charge);
(4) an electrode area; and
(5) a positive-electrode application quantity.

7. The method of diagnosing a secondary battery according to claim 1 or 2, further comprising:

estimating a threshold value of electrolyte diffusion coefficient of the secondary battery being diagnosed, Dth, using a fourth trained model adapted to estimate the threshold value of electrolyte diffusion coefficient, that is the value at which the secondary battery becomes unsuitable for reuse, based on predetermined input data; and calculating a difference between the threshold value Dth and the electrolyte diffusion coefficient Dn at the time of diagnosis, $\Delta D$,
wherein the input data for the fourth trained model contains the following items, (1) to (5):

(1) a discharge capacity measured at a discharge rate equivalent to that for the measurement of the rated capacity;
(2) a magnitude of electric current equal to that of item (3) of the input data for the first trained model;
(3) a voltage drop as determined at a predetermined period of time from initiation of discharge (or a voltage rise as determined at a predetermined period of time from initiation of charge);
(4) an electrode area; and
(5) a positive-electrode application quantity.

8. A method of diagnosing a secondary battery comprising:
estimating a difference $\Delta D$ for a secondary battery being diagnosed using a fifth trained model adapted to estimate the difference $\Delta D$ between an electrolyte diffusion coefficient of the secondary battery and a threshold value of electrolyte diffusion coefficient, that is the value at which the secondary battery becomes unsuitable for reuse, based on input data containing items (1) to (8) provided below:

(1) a rated capacity (initial capacity) of the secondary battery;
(2) a discharge capacity measured at a discharge rate equivalent to that for a measurement of the rated capacity; and
(3) a magnitude of electric current from measurements of the following items, (4) to (6):
(4) a voltage drop as determined at a predetermined period of time from initiation of discharge (or a voltage rise as determined at a predetermined period of time from initiation of charge);
(5) a voltage drop as determined at a predetermined SOC;
(6) a temperature rise as determined at a predetermined SOC;
(7) an electrode area; and
(8) a positive-electrode application quantity.

9. The method of diagnosing a secondary battery according to claim 8, wherein the input data for the fifth trained model further contains one or more selected from the following items, (9) to (14):

(9) a discharge capacity measured at the electric current of item (3) above;
(10) a type of a positive-electrode active material;
(11) a porosity of a positive-electrode mixture layer (or a density of a positive-electrode mixture layer);
(12) a type of a negative-electrode active material;
(13) a porosity of a negative-electrode mixture layer (or a density of a negative-electrode mixture layer); and
(14) a thermal capacity of the secondary battery.

10. A program for diagnosing a secondary battery adapted to cause a computer to perform:
estimating an electrolyte diffusion coefficient at a time of diagnosis of a secondary battery being diagnosed, Dn, using a first trained model adapted to estimate the electrolyte diffusion coefficient of the secondary battery based on input data containing the following items, (1) to (6):

(1) a rated capacity (initial capacity) of the secondary battery;
(2) a discharge capacity measured at a discharge rate equivalent to that for a measurement of the rated capacity; and
(3) a magnitude of electric current for measurements of the following items, (4) to (6):
(4) a voltage drop as determined at a predetermined period of time from initiation of discharge (or a voltage rise as determined at a predetermined period of time from initiation of charge);
(5) a voltage drop as determined at a predetermined SOC; and
(6) a temperature rise as determined at a predetermined SOC.

11. A program for diagnosing a secondary battery adapted to cause a computer to perform:
estimating a difference $\Delta D$ for a secondary battery being diagnosed using a fifth trained model adapted to estimate the difference $\Delta D$ between an electrolyte diffusion coefficient of the secondary battery and a threshold value of electrolyte diffusion coefficient, that is the value at which the secondary battery becomes unsuitable for reuse, based on input data containing items (1) to (8) provided below:

(1) a rated capacity (initial capacity) of the secondary battery;
(2) a discharge capacity measured at a discharge rate equivalent to that for a measurement of the rated capacity; and
(3) a magnitude of electric current for measurements of the following items, (4) to (6):
(4) a voltage drop as determined at a predetermined period of time from initiation of discharge (or a voltage rise as determined at a predetermined period of time from initiation of charge);
(5) a voltage drop as determined at a predetermined SOC;
(6) a temperature rise as determined at a predetermined SOC;
(7) an electrode area; and
(8) a positive-electrode application quantity.

12. An apparatus for diagnosing a secondary battery comprising:
an estimation device adapted to estimate an electrolyte diffusion coefficient at a time of diagnosis of a secondary battery being diagnosed, Dn, using a first trained model adapted to estimate the electrolyte diffusion coefficient of the secondary battery based on input data containing the following items, (1) to (6):

(1) a rated capacity (initial capacity) of the secondary battery;
(2) a discharge capacity measured at a discharge rate equivalent to that for a measurement of the rated capacity; and
(3) a magnitude of electric current for measurements of the following items, (4) to (6):
(4) a voltage drop as determined at a predetermined period of time from initiation of discharge (or a voltage rise as determined at a predetermined period of time from initiation of charge);
(5) a voltage drop as determined at a predetermined SOC; and
(6) a temperature rise as determined at a predetermined SOC.

13. An apparatus for diagnosing a secondary battery comprising:
an estimation device adapted to estimate a difference ΔD for a secondary battery being diagnosed using a fifth trained model adapted to estimate the difference ΔD between an electrolyte diffusion coefficient of the secondary battery and a threshold value of electrolyte diffusion coefficient, that is the value at which the secondary battery becomes unsuitable for reuse, based on input data containing items (1) to (8) provided below:

(1) a rated capacity (initial capacity) of the secondary battery;
(2) a discharge capacity measured at a discharge rate equivalent to that for a measurement of the rated capacity; and
(3) a magnitude of electric current for measurements of the following items, (4) to (6):
(4) a voltage drop as determined at a predetermined period of time from initiation of discharge (or a voltage rise as determined at a predetermined period of time from initiation of charge);
(5) a voltage drop as determined at a predetermined SOC;
(6) a temperature rise as determined at a predetermined SOC;
(7) an electrode area; and
(8) a positive-electrode application quantity.

*Fig.1*

```
            ( START )
                │
                ▼
┌──────────────────────────────────────┐
│   ESTIMATE CHARACTERISTIC PARAMETERS  │──── SA1
│        AT TIME OF DIAGNOSIS           │
└──────────────────────────────────────┘
                │
                ▼
┌──────────────────────────────────────┐
│   OBTAIN RELATIONSHIP BETWEEN ELECTROLYTE  │──── SA2
│ DIFFUSION COEFFICIENT AND DISCHARGE CAPACITY │
└──────────────────────────────────────┘
                │
                ▼
┌──────────────────────────────────────┐
│        DETERMINE THRESHOLD VALUE      │──── SA3
│   Dth OF ELECTROLYTE DIFFUSION COEFFICIENT │
└──────────────────────────────────────┘
                │
                ▼
┌──────────────────────────────────────┐
│         OBTAIN ΔD = Dn − Dth          │──── SA4
└──────────────────────────────────────┘
                │
                ▼
             ( END )
```

*Fig.2*

```
            ( START SA1 )
                │
                ▼
┌──────────────────────────────────────┐
│      INPUT BASIC SPECIFICATIONS OF    │──── SA1-1
│        BATTERY BEING DIAGNOSED        │
└──────────────────────────────────────┘
                │
                ▼
┌──────────────────────────────────────┐
│      INPUT DATA OBTAINED BY MEASURING │──── SA1-2
│          LOAD CHARACTERISTICS         │
└──────────────────────────────────────┘
                │
                ▼
┌──────────────────────────────────────┐
│       ESTIMATE STATIC PARAMETERS OF   │──── SA1-3
│         BATTERY BEING DIAGNOSED       │
└──────────────────────────────────────┘
                │
                ▼
┌──────────────────────────────────────┐
│      ESTIMATE DYNAMIC PARAMETERS OF   │──── SA1-4
│         BATTERY BEING DIAGNOSED       │
└──────────────────────────────────────┘
                │
                ▼
            ( END SA1 )
```

*Fig.3*

*Fig.4*

*Fig.5*

```
                    ┌─────────┐
                    │  START  │
                    └─────────┘
                         │
                         ▼
┌──────────────────────────────────────────────┐
│   ESTIMATE Dn USING TRAINED MODEL A           │── S1
└──────────────────────────────────────────────┘
                         │
                         ▼
┌──────────────────────────────────────────────┐
│   ESTIMATE Dth USING TRAINED MODEL B          │── S2
└──────────────────────────────────────────────┘
                         │
                         ▼
┌──────────────────────────────────────────────┐
│   CALCULATE  △D = Dn − Dth                     │── S3
└──────────────────────────────────────────────┘
                         │
                         ▼
                    ┌─────────┐
                    │   END   │
                    └─────────┘
```

*Fig.6*

```
                    ┌─────────┐
                    │  START  │
                    └─────────┘
                         │
                         ▼
┌──────────────────────────────────────────────┐
│   ESTIMATE Dn USING TRAINED MODEL A           │── S1
└──────────────────────────────────────────────┘
                         │
                         ▼
┌──────────────────────────────────────────────┐
│   ESTIMATE σ USING TRAINED MODEL C            │── S4
└──────────────────────────────────────────────┘
                         │
                         ▼
┌──────────────────────────────────────────────┐
│   ESTIMATE Dth USING TRAINED MODEL B          │── S2
└──────────────────────────────────────────────┘
                         │
                         ▼
┌──────────────────────────────────────────────┐
│   CALCULATE  △D = Dn − Dth                     │── S3
└──────────────────────────────────────────────┘
                         │
                         ▼
                    ┌─────────┐
                    │   END   │
                    └─────────┘
```

*Fig.7*

```
         ┌─────────┐
         │  START  │
         └────┬────┘
              │
   ┌──────────▼───────────────────────────┐
   │ ESTIMATE Dn USING TRAINED MODEL A     │──S1
   └──────────┬───────────────────────────┘
              │
   ┌──────────▼───────────────────────────┐
   │ ESTIMATE Dth USING TRAINED MODEL D    │──S5
   └──────────┬───────────────────────────┘
              │
   ┌──────────▼───────────────────────────┐
   │ CALCULATE △D = Dth − Dth              │──S3
   └──────────┬───────────────────────────┘
              │
         ┌────▼────┐
         │   END   │
         └─────────┘
```

*Fig.8*

```
         ┌─────────┐
         │  START  │
         └────┬────┘
              │
   ┌──────────▼───────────────────────────┐
   │ ESTIMATE △D USING TRAINED MODEL E     │──S6
   └──────────┬───────────────────────────┘
              │
         ┌────▼────┐
         │   END   │
         └─────────┘
```

*Fig.9*

*Fig.10*

| NORMALIZE ALL DATA |
|---|

↓

| LEARN USING NONLINEAR SVR |
|---|

↓

| STORE TRAINED MODEL |
|---|

*Fig.11*

$y=0.9837x$
$R^2=0.9953$

TRAINING DATA

PREDICTED VALUE OF Dth ($\times 10^{-6}$ cm$^2$/sec.)

VALUE OF Dth FROM ANALYSIS ($\times 10^{-6}$ cm$^2$/sec.)

*Fig.12*

$y=0.9885x$
$R^2=0.9987$

VALIDATION DATA

PREDICTED VALUE OF Dth ($\times 10^{-6}$ cm$^2$/sec.)

VALUE OF Dth FROM ANALYSIS ($\times 10^{-6}$ cm$^2$/sec.)

*Fig.13*

*Fig.14*

*Fig.15*

*Fig.16*

*Fig.17*

*Fig.18*

## INTERNATIONAL SEARCH REPORT

| International application No. |
| --- |
| **PCT/JP2024/013484** |

| A. | CLASSIFICATION OF SUBJECT MATTER |
| --- | --- |

*H01M 10/48*(2006.01)i; *G01R 31/367*(2019.01)i; *G01R 31/382*(2019.01)i; *G01R 31/385*(2019.01)i; *H02J 7/00*(2006.01)i
FI:  H01M10/48 P; H01M10/48 301; G01R31/367; G01R31/382; G01R31/385; H02J7/00 Q

According to International Patent Classification (IPC) or to both national classification and IPC

| B. | FIELDS SEARCHED |
| --- | --- |

Minimum documentation searched (classification system followed by classification symbols)

H01M10/48: G01R31/367; G01R31/382; G01R31/385; H02J7/00

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

| C. | DOCUMENTS CONSIDERED TO BE RELEVANT |
| --- | --- |

| Category* | Citation of document, with indication, where appropriate, of the relevant passages | Relevant to claim No. |
| --- | --- | --- |
| A | US 2020/0074297 A1 (LG ELECTRONICS INC.) 05 March 2020 (2020-03-05)<br>entire text, all drawings | 1-13 |
| A | US 2016/0131715 A1 (HYUNDAI MOTOR CO.) 12 May 2016 (2016-05-12)<br>entire text, all drawings | 1-13 |
| A | JP 2013-113625 A (TOYOTA JIDOSHA KABUSHIKI KAISHA) 10 June 2013 (2013-06-10)<br>entire text, all drawings | 1-13 |
| A | JP 2013-044580 A (TOYOTA JIDOSHA KABUSHIKI KAISHA) 04 March 2013<br>(2013-03-04)<br>entire text, all drawings | 1-13 |
| A | JP 2013-046446 A (TOYOTA JIDOSHA KABUSHIKI KAISHA) 04 March 2013<br>(2013-03-04)<br>entire text, all drawings | 1-13 |

☐ Further documents are listed in the continuation of Box C.    ☑ See patent family annex.

| * | Special categories of cited documents: | "T" | later document published after the international filing date or priority date and not in conflict with the application but cited to understand the principle or theory underlying the invention |
| --- | --- | --- | --- |
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | "&" | document member of the same patent family |
| "P" | document published prior to the international filing date but later than the priority date claimed | | |

| Date of the actual completion of the international search | Date of mailing of the international search report |
| --- | --- |
| **03 June 2024** | **11 June 2024** |

| Name and mailing address of the ISA/JP | Authorized officer |
| --- | --- |
| **Japan Patent Office (ISA/JP)**<br>**3-4-3 Kasumigaseki, Chiyoda-ku, Tokyo 100-8915**<br>**Japan** | |
| | Telephone No. |

Form PCT/ISA/210 (second sheet) (July 2022)

**INTERNATIONAL SEARCH REPORT**
Information on patent family members

International application No.

**PCT/JP2024/013484**

| Patent document cited in search report | | | Publication date (day/month/year) | Patent family member(s) | | | Publication date (day/month/year) |
|---|---|---|---|---|---|---|---|
| US | 2020/0074297 | A1 | 05 March 2020 | WO | 2020/050599 | A1 | |
| | | | | KR | 10-2020-0026607 | A | |
| US | 2016/0131715 | A1 | 12 May 2016 | US | 2019/0250215 | A1 | |
| | | | | KR | 10-1619634 | B1 | |
| | | | | CN | 106033112 | A | |
| JP | 2013-113625 | A | 10 June 2013 | (Family: none) | | | |
| JP | 2013-044580 | A | 04 March 2013 | (Family: none) | | | |
| JP | 2013-046446 | A | 04 March 2013 | (Family: none) | | | |

Form PCT/ISA/210 (patent family annex) (July 2022)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2017097997 A
- JP 2023003516 W
- WO 2023149532 A

- JP 2023027999 W
- WO 2024029501 A

**Non-patent literature cited in the description**

- **MARC DOYLE et al.** Modeling of Galvanostatic Charge and Discharge of the Lithium/Polymer/Insertion Cell. *J. Electrochem. Soc.*, June 1993, vol. 140 (6)